# EUROPÄISCHE PATENTSCHRIFT

(11) **EP 1 140 824 B1**
(45) Veröffentlichungstag und Bekanntmachung des Hinweises auf die Patenterteilung: **19.05.2004**
(21) Anmeldenummer: 99963352.2
(22) Anmeldetag: 01.12.1999
(51) Int. Cl.: C07D 209/10, C07D 209/12

(54) **VERFAHREN ZUR HERSTELLUNG VON 3-ALKANOYL- UND 3-ALKYLINDOLEN**
METHOD FOR PRODUCING 3-ALKANOYLINDOLES AND 3-ALKYLINDOLES
PROCEDE DE PRODUCTION DE 3-ALCANOYLINDOLS ET DE 3-ALKYLINDOLS

(30) Priorität: 17.12.1998 DE 19858340
(43) Veröffentlichungstag der Anmeldung: 10.10.2001
(73) Patentinhaber: MERCK PATENT GmbH, 64293 Darmstadt (DE)
(72) Erfinder: BATHE, Andreas, D-64283 Darmstadt (DE); TILLY, Herbert, D-64319 Pfungstadt (DE)
(86) Internationale Anmeldenummer: PCT/EP1999/009334
(87) Internationale Veröffentlichungsnummer: WO 2000/035872

(56) Entgegenhaltungen:
- DE-A- 2 524 299
- H BÖTTCHER, R.GERICKE: "Synthese von 3-[4-(1,2,3,6-Tetrahydro-4-phenyl-1-pyridy l)butyl]-5-indolcarbonsäure, eine blutdrucksenkende Verbindung mit neuartigem wirkprinzip" LIEBIEGS ANN. CHEM.,1988, Seiten 749-752, XP000882712 in der Anmeldung erwähnt
- SNIDER, BARRY B. ET AL: "Use of ethylaluminum dichloride as a catalyst for the Friedel - Crafts acylation of alkenes" J. ORG. CHEM. , Bd. 47, Nr. 27, 1982, Seiten 5393-5395, XP000881554
- EISCH, JOHN J. ET AL: "Reductive deoxygenation of ketones and secondary alcohols by organoaluminum Lewis acids" J. ORG. CHEM. , Bd. 57, Nr. 7, 1992, Seiten 2143-2147, XP000881555
- ATUL AGARWAL ET AL.: "A new synthesis of the potent 5-HT1 receptor ligand, 5-carboxyamidotryptamine (5-CT)" SYNTHETIC COMMUNICATIONS, Bd. 23, Nr. 8, 1993, Seiten 1101-1110, XP000881512 in der Anmeldung erwähnt
- CHENGXI YANG ET AL.: "The use of Lewis acid in the reaction of zinc salts of indoles and acyl chloride" SYNTHETIC COMMUNICATIONS, Bd. 27, Nr. 12, 1997, Seiten 2125-2132, XP000881533

## Beschreibung

Die Erfindung betrifft ein Verfahren zur Herstellung von Verbindungen der Formel I 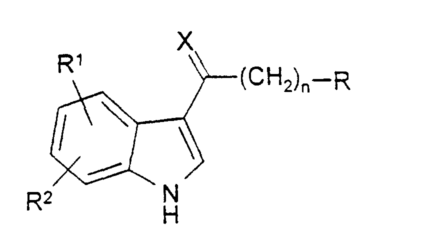 worin
- R: Hal oder Methyl,
- R¹, R²: jeweils unabhängig voneinander H, A', Aryl, NH₂, NHA", N(A")₂, COOA"', CN oder Hal,
- X: O oder H,H,
- A', A", A"': jeweils unabhängig voneinander Alkyl mit 1-6 C-Atomen,
- Hal: F, Cl, Br oder I und
- n: 1, 2, 3, 4, 5 oder 6
bedeuten,
sowie ihrer Säureadditionssalze, dadurch gekennzeichnet, daß man,
a) falls X O bedeutet und R, R¹, R² und n die angegebenen Bedeutungen haben,
   eine Verbindung der Formel II 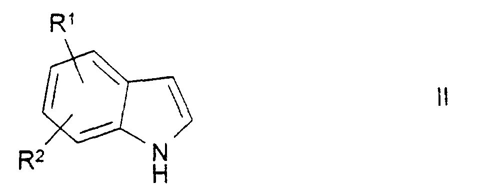 worin
   - R¹, R²: jeweils unabhängig voneinander H, A', Aryl, NH₂, NHA", N(A")₂, COOA"', CN oder Hal,
   - A', A'', A''': jeweils unabhängig voneinander Alkyl mit 1-6 C-Atomen und
   - Hal: F, Cl, Br oder I
   bedeuten,
   mit einer Verbindung der Formel III

   R-(CH₂)ₙ-CO-L III

   worin
   - R: Hal oder Methyl,
   - L: Cl, Br, I, OH oder eine freie oder reaktionsfähig funktionell abgewandelte OH-Gruppe bedeutet,
   - Hal: F, Cl, Br oder I und
   - n: 1, 2, 3, 4, 5 oder 6
   bedeuten,
   in einer Friedel-Crafts Acylierung unter Katalyse von Lewis-sauren Metallhalogeniden des Typs R'-Al(Cl)₂,
   worin
   - R': A oder Aryl',
   - A: Alkyl mit 1-6 C-Atomen,
   - Aryl': unsubstituiertes oder ein- oder zweifach durch A', OA' oder Hal substituiertes Phenyl,
   - Hal: F oder Cl
   bedeuten,
   umsetzt,
   oder
b) falls X H,H bedeutet und R, R¹, R² und n die angegebenen Bedeutungen haben,
   eine Verbindung der Formel I, worin X O bedeutet und R, R¹, R² und n die angegebenen Bedeutungen haben,
   mit komplexen Hydriden unter Aktivierung durch Lewis-saure Metallhalogenide des Typs R'-Al(Cl)₂,
worin
- R': A oder Aryl',
- A: Alkyl mit 1-6 C-Atomen,
- Aryl': unsubstituiertes oder ein- oder zweifach durch A', OA' oder Hal substituertes Phenyl,
- Hal: F oder Cl
bedeuten,
reduziert,
und/oder daß man eine erhaltene Base der Formel I durch Behandeln mit einer Säure in eines ihrer Säureadditionssalze umwandelt.

Bekannt sind Verfahren zur Herstellung von acylierten Indolen z.B. beschrieben von M. Tani et al., Chem. Pharm. Bull. **38** (12) 3261-3267 (1990), wobei der Indolring in der 2-Position durch Ethoxycarbonyl substituiert ist.

Ein Verfahren zur Herstellung von Methyl-3-(4-chlor-1-oxobutyl)-5-indolcarboxylat unter AlCl₃-Katalyse ist von Böttcher et al. in Liebigs Ann. Chem. **1988,** 749-752 beschrieben.

In J. Med. Chem. **1980,** 23, 1306-1310 ist eine Indolacylierung über intermediär gebildete MgX-Salze von Indol mit R-CO-X von C. Gueremy beschrieben.

In Tetrahedron Letters **28** (32), 3741-3744 (1987) ist auch eine Indolacylierung über intermediär gebildete MgX-Salze von J. Bergman et al. beschrieben.

Eine andere Acetylierung von 5-Cyanindol mit Acetylchlorid unter Katalyse von SnCl₄ ist von Agarwal et al. in Synthetic Communications **23** (8), 1101-1110 (1993) beschrieben.

Die Reduktion von 4-Indol-3-yl-4-oxobuttersäure mit LiAlH₄ ist von J.S.L. Ibaceta-Lizana in J. Chem. Soc. Perkin Trans. II 1987, 1221-1226 beschrieben.

Die Reduktion eines 3-Alkanoylindolesters mit NaBH₄/BF₃-Ether ist von Böttcher et al. in Liebigs Ann. Chem. **1988,** 749-752 beschrieben.

Eine andere Reduktion eines Phthalimidderivates von 3-Acetyl-5-cyanindol mit NaBH₄ unter Isopropanol-Katalyse ist von Agarwal et al. in Synthetic Communications **23** (8), 1101-1110 (1993) beschrieben.

Überraschenderweise ergaben Untersuchungen im Rahmen der Synthese von Arzneimitteln, welche z.B. in der DE 43 33 254 (EP 0 648 767) beschrieben sind, daß die Verbindungen der Formel I im Vergleich zum Stand der Technik in wenigstens vergleichbarer oder höherer Ausbeute erhalten werden können, wobei als entscheidende Vorteile dabei die einfache und in homogener Phase durchzuführende Reaktion sowie eine dadurch bedingt einfache Produktisolierung zu nennen sind.
Das bedeutet in der Konsequenz auch einen geringeren Lösungsmittelund Energieverbrauch.
So kann zur Herstellung von Verbindungen der Formel I, worin X O bedeutet, bei der Acylierung gemäß Schritt a) als Katalysator z.B. das flüssige Isobutyl-aluminiumdichlorid (i-Bu-AlCl₂) per Pumpe unverdünnt verwendet werden. Die aus dem Stand der Technik bekannte, oftmals unter AlCl₃-Katalyse ausgelöste Bildung schwerlöslicher und unrührbarer Feststoffanteile, erfolgt nicht. Als weiterer Vorteil ist das Auftreten von weniger Nebenprodukten zu nennen, da sich z.B. das genannte i-Bu-AlCl₂ als schwächere Lewis-Säure als AlCl₃ verhält, und eine Aktivierung einer Chloralkyl-Funktion in der Seitenkette und eine dadurch abgeleitete Friedel-Crafts-Alkylierung als Nebenreaktion stark unterdrückt wird.

Auch bei der erfindungsgemäßen Reduktion gemäß Schritt b) der Verbindungen der Formel I, worin X O bedeutet, zu den Verbindungen der Formel I, worin X H,H bedeutet, sind als Vorteile zum Stand der Technik vergleichbare oder höhere Ausbeuten bei leichterer Reaktionsführung und Produktisolierung zu nennen. Als weiterer Vorteil ist auch hier das Auftreten von weniger Nebenprodukten zu nennen, insbesondere dann, wenn sich in den Positionen 4 bis 7 des lndols reduktionsempfindliche Substituenten wie CN oder Estergruppen befinden.

Nach dem erfindungsgemäßen Verfahren wird insbesondere z.B. die Verbindung 3-(4-Chlorbutanoyl)-indol-5-carbonitril hergestellt, die anschließend zu der in der DE 43 33 254 offenbarten Verbindung 1-[4-(5-Cyan-indol-3-yl)-butyl]-4-(2-carbamoyl-benzofuran-5-yl)-piperazin umgesetzt wird.

Die Erfindung betrifft daher insbesondere ein Verfahren zur Herstellung von Verbindungen der Formel I 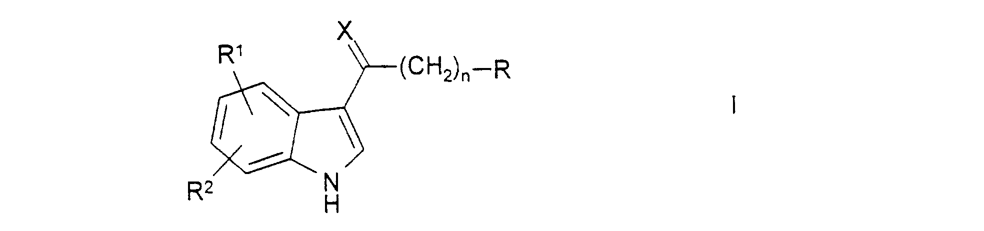 worin
- R: Hal,
- R¹: H,
- R²: CN
- X: O oder H,H,
- Hal: F, Cl, Br oder I und
- n: 2, 3 oder 4
bedeuten,
sowie ihrer Säureadditionssalze, dadurch gekennzeichnet, daß man,
a) falls X O bedeutet und R, R¹, R² und n die angegebenen Bedeutungen haben,
   eine Verbindung der Formel II 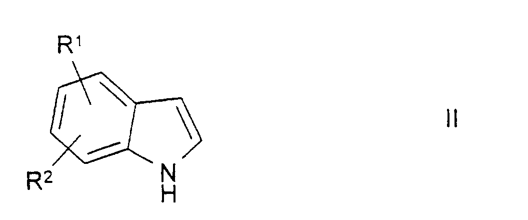 worin
   - R¹: H und
   - R²: CN,
   bedeuten,
   mit einer Verbindung der Formel III

   R-(CH₂)ₙ-CO-L III

   worin
   - R: Hal,
   - L: Cl, Br, I, OH oder eine freie oder reaktionsfähig funktionell abgewandelte OH-Gruppe bedeutet,
   - Hal: F, Cl, Br, I und
   - n: 2, 3 oder 4
   bedeuten,
   in einer Friedel-Crafts Acylierung unter Katalyse von Lewis-sauren Metallhalogeniden des Typs R'-Al(Cl)₂,
   worin
   - R': A,
   - A: Alkyl mit 1-6 C-Atomen,
   bedeuten,
   umsetzt,
   oder
b) falls X H,H bedeutet und R, R¹, R² und n die angegebenen Bedeutungen haben, eine Verbindung der Formel I, worin X O bedeutet und R, R¹, R² und n die angegebenen Bedeutungen haben,
   mit komplexen Hydriden unter Aktivierung durch Lewis-saure Metallhalogenide des Typs R'-Al(Cl)₂,
worin
- R': A,
- A: Alkyl mit 1-6 C-Atomen,
bedeuten,
reduziert,
und/oder daß man eine erhaltene Base der Formel I durch Behandeln mit einer Säure in eines ihrer Säureadditionssalze umwandelt.

Die Verbindungen der Formel I, worin X O bedeutet und die in Schritt b) reduziert werden, können grundsätzlich auch nach üblichen Verfahren durch Acylierung unter z.B. AlCl₃-Katalyse erhalten werden.
Vorzugsweise werden sie jedoch gemäß Reaktionsschritt a) hergestellt und anschließend gemäß Schritt b) reduziert.

Gegenstand der Erfindung ist daher vorzugsweise ein Verfahren nach den beiden genannten Verfahren, zur Herstellung von Verbindungen gemäß Formel I, worin X H,H bedeutet und R, R¹, R² und n die angegebenen Bedeutungen haben, dadurch gekennzeichnet, daß man die Verbindungen der Formel I, worin X O bedeutet und R, R¹, R² und n die angegebenen Bedeutungen haben, nach Schritt a) herstellt und anschließend nach Schritt b) reduziert.

A', A" und A''' bedeuten Alkyl mit 1, 2, 3, 4, 5 oder 6 C-Atomen, vorzugsweise mit 1, 2, 3 oder 4 C-Atomen, insbesonders sind bevorzugt z.B. Methyl oder Ethyl, weiterhin Propyl, Isopropyl, ferner auch Butyl, Isobutyl, sek.-Butyl oder tert.-Butyl.

R bedeutet in den Verbindungen der Formeln I und III vorzugsweise Cl oder Methyl.

In den Verbindungen der Lewis-sauren Metallhalogenide des Typs R'-Al(Cl)₂ bedeutet R' vorzugsweise Methyl, Ethyl, Propyl, Isopropyl, Butyl, tert.-Butyl, Isobutyl, Pentyl, Neopentyl, Isopentyl, Phenyl, o-, m- oder p-Tolyl, o-, m- oder p-Methoxyphenyl, o-, m- oder p-Fluor, o-, m- oder p-Chlorphenyl. Ganz besonders bevorzugt bedeutet R' Isopropyl oder Isobutyl.
Die bevorzugte Verbindung Isobutyl-Al(Cl)₂ ist z.B. aus der Polymerchemie bekannt.

Aryl bedeutet in den Verbindungen der Formeln I und II unsubstituiertes oder ein- oder zweifach durch A, OA oder Hal substituiertes Phenyl.

R¹ und R² bedeuten in den Verbindungen der Formeln I und II vorzugsweise, jeweils unabhängig voneinander, H, Methyl, Ethyl, Propyl, Phenyl, Amino, Methylamino, Ethylamino, Dimethylamino, Diethylamino, Methoxycarbonyl, Ethoxycarbonyl, Cyan, Fluor oder Chlor, ferner auch Carboxy.
Ganz besonders bevorzugt bedeutet R¹ H und R² Cyan.

n bedeutet in den Verbindungen der Formel I und III vorzugsweise 2, 3 oder 4, insbesondere 2 oder 3.

Die Verbindungen der Formel II und III sind größtenteils bekannt.
In den Verbindungen der Formel III bedeutet der Rest L vorzugsweise Cl oder Br; er kann jedoch auch I, OH oder eine reaktionsfähig abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl-, p-Tolylsulfonyloxy, 1- oder 2-Naphthalinsulfonyloxy) bedeuten. L kann auch ein geeignetes Anhydrid sein.

Die Verbindungen der Formel II und III werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Umsetzung der Verbindungen II und III verläuft in einem geeigneten Lösungsmittel. Als Lösungsmittel eignen sich z.B. Kohlenwasserstoffe, wie Benzol, Toluol, Xylol; chlorierte Kohlenwasserstoffe wie z.B. Dichlormethan; Ketone wie Aceton, Butanon; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander.
Die Reaktionszeit liegt je nach den angewendeten Bedingungen zwischen einigen Minuten und 14 Tagen, die Reaktionstemperatur zwischen etwa 0° und 150°, normalerweise zwischen 0° und 60°.

Die Reduktion der Verbindungen der Formel I, worin X O bedeutet, erfolgt mit komplexen Hydriden unter Aktivierung von Lewis-Säuren, in einem geeigneten Lösungsmittel. Als Lösungsmittel eignen sich z.B. Kohlenwasserstoffe, wie Benzol, Toluol, Xylol; chlorierte Kohlenwasserstoffe wie z.B. Dichlormethan; Ketone wie Aceton, Butanon; Ether wie Tetrahydrofuran (THF) oder Dioxan; Amide wie Dimethylformamid (DMF) oder N-Methylpyrrolidon; Nitrile wie Acetonitril, gegebenenfalls auch Gemische dieser Lösungsmittel untereinander.
Als komplexe Hydride sind Verbindungen vom Typ MBH₄ bevorzugt, mit M = z.B. Na, Li, oder 0,5 Ca.
Die Reaktionszeit liegt je nach den angewendeten Bedingungen zwischen einigen Minuten und 14 Tagen, die Reaktionstemperatur zwischen etwa 0° und 150°, normalerweise zwischen 0° und 60°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B.

Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Beispiel 1

### Indol-5-carbonitril -> 3-(4-Chlorbutanoyl)-indol-5-carbonitril

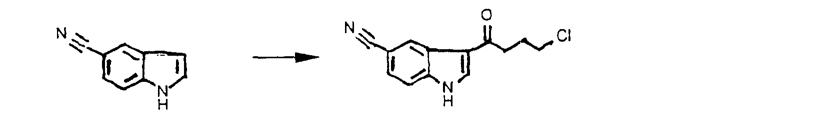

### Versuchsbeschreibung

Indol-5-carbonitril (4800 g) wird unter Schutzgas Stickstoff in Dichlormethan (70 L) unter Rühren bei 0-10 °C gelöst und mit Cl-(CH₂)₃COCl (6640 g) versetzt. Es folgt die T-kontrollierte Zugabe (0-10 °C) von Isobutylaluminiumdichlorid (7300 g). Nach Ende der Acylierung (erkenntlich durch chrom. Analyse) wird die Mischung auf Eis/Wasser (64 Kg) gegeben und das kristalline Rohprodukt 3-(4-Chlorbutanoyl)-indol-5-carbonitril abgetrennt. Zu Reinigung wird das Keton kristallisiert (6940 g / 82%)

### Indol-5-carbonitril -> 3-(3-Chlorpropanoyl)-indol-5-carbonitril

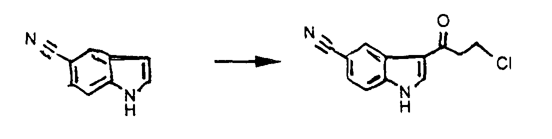

### Versuchsbeschreibung

Indol-5-carbonitril (57.0 g) wird unter Schutzgas Stickstoff in Dichlormethan (790 g) unter Rühren bei 0-10 °C gelöst und mit Cl-(CH₂)₂COCl (61 g) versetzt. Es folgt die T-kontrollierte Zugabe (0-10 °C) von Isobutylaluminiumdichlorid (124 g) . Nach Ende der Acylierung (erkenntlich durch chrom. Analyse) wird die Mischung auf Eis/Wasser gegeben und das kristalline 3-(3-Chlorpropanoyl)-indol-5-carbonitril abgetrennt und i.Vak. getrocknet (ca. 83 g / 89%).

### Beispiel 2

### 3-(4-Chlorbutanoyl)-indol-5-carbonitril -> 3-(4-Chlorbutyl)-indol-5-carbonitril

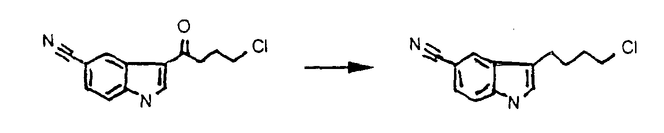

### Versuchsbeschreibung

3-(4-Chlorbutanoyl)-indol-5-carbonitril (75.5 g) wird unter Schutzgas Stickstoff unter Rühren in Dichlormethan (1980 g) bei 0-10 °C gelöst und mit NaBH₄ (46.3 g) versetzt. Es folgt die T-kontrollierte Zugabe (0-10 °C) von Isobutylaluminiumdichlorid (190 g). Nach Ende der Reduktion (erkenntlich durch chrom. Analyse) wird die Mischung auf Eis/Wasser gegeben und das kristalline Produkt 3-(4-Chlorbutyl)-indol-5-carbonitril als einheitliches Material abgetrennt (68g; 95%).

### 3-(3-Chlorpropanoyl)-indol-5-carbonitril -> 3-(3-Chlorpropyl)-indol-5-carbonitril

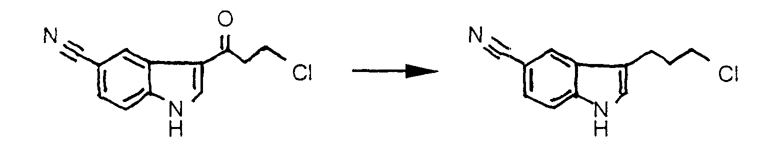

### Versuchsbeschreibung

3-(3-Chlorpropanoyl)-indol-5-carbonitril (4.8 g) wird unter Schutzgas Stickstoff unter Rühren in Dichlormethan (224 g) bei 0-10 °C gelöst und mit NaBH₄ (3.1 g) versetzt. Es folgt die T-kontrollierte Zugabe (0-10 °C) von Isobutylaluminiumdichlorid (13 g) . Nach Ende der Reduktion (erkenntlich durch chrom. Analyse) wird die Mischung auf Eis/Wasser gegeben und das kristalline Rohprodukt 3-(3-Chlorpropyl)-indol-5-carbonitril abgetrennt und i.Vak. getrocknet. Zu Reinigung wird das Indol kristallisiert (3.9 g; 87%).

### Vergleichsversuch 1

### 3-(4-Chlorbutanoyl)-indol-5-carbonitril -> 3-(4-Chlorbutyl)-indol-5-carbonitril

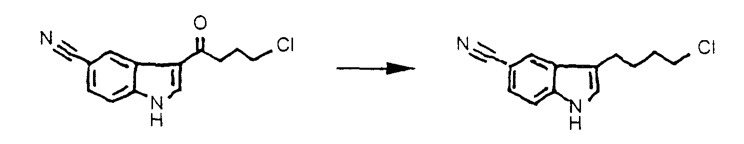

### Versuchsbeschreibung

3-(4-Chlorbutanoyl)-indol-5-carbonitril (75.5 g) wird unter Schutzgas Stickstoff unter Rühren in Dichlormethan (1980 g) bei 0-10 °C gelöst und mit LiAlH₄ (46 g) versetzt. Nach üblicher Reaktionszeit und Aufarbeitung konnte kein Produkt isoliert werden.

### Vergleichsversuch 2

### 3-(4-Chlorbutanoyl)-indol-5-carbonitril -> 3-(4-Chlorbutyl)-indol-5-carbonitril

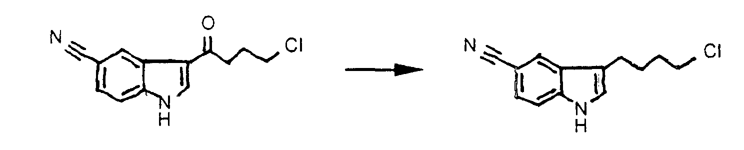

### Versuchsbeschreibung

3-(4-Chlorbutanoyl)-indol-5-carbonitril (75.5 g) wird unter Schutzgas Stickstoff unter Rühren in Dichlormethan (1980 g) bei 0-10 °C gelöst und mit NaBH₄/BF₃-Ether versetzt. Nach üblicher Reaktionszeit und Aufarbeitung konnte kein Produkt isoliert werden.

## Patentansprüche

1. Verfahren zur Herstellung von Verbindungen der Formel I 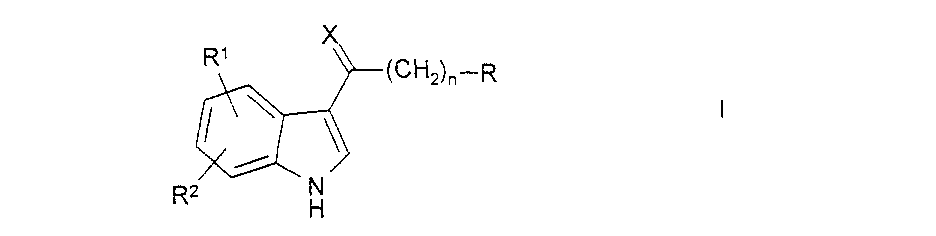 worin
R Hal oder Methyl,
R¹, R² jeweils unabhängig voneinander H, A', Aryl, NH₂, NHA", N(A")₂, COOA"', CN oder Hal,
X O oder H,H,
A', A", A"' jeweils unabhängig voneinander Alkyl mit 1-6 C-Atomen
Hal F, Cl, Br oder I und
n 1, 2, 3, 4, 5 oder 6
bedeuten,
sowie ihrer Säureadditionssalze, **dadurch gekennzeichnet, daß** man,
a) falls X O bedeutet und R, R¹, R² und n die angegebenen Bedeutungen haben,
eine Verbindung der Formel II 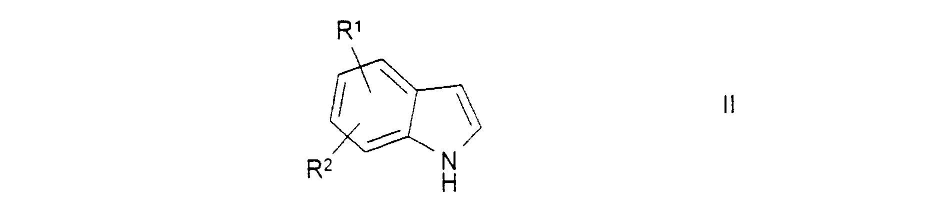 worin
R¹, R² jeweils unabhängig voneinander H, A', Aryl, NH₂, NHA", N(A")₂, COOA"', CN oder Hal,
A', A", A"' jeweils unabhängig voneinander Alkyl mit 1-6 C-Atomen und
Hal F, Cl, Br oder l
bedeuten,
mit einer Verbindung der Formel III
R-(CH₂)ₙ-CO-L III
worin
R Hal oder Methyl,
L Cl, Br, I, OH oder eine freie oder reaktionsfähig funktionell abgewandelte OH-Gruppe bedeutet,
Hal F, Cl, Br oder I und
n 1, 2, 3, 4, 5 oder 6
bedeuten,
in einer Friedel-Crafts Acylierung unter Katalyse von Lewis-sauren Metallhalogeniden des Typs R'-Al(Cl)₂,
worin
R' A oder Aryl',
A Alkyl mit 1-6 C-Atomen,
Aryl' unsubstituiertes oder ein- oder zweifach durch A', OA' oder Hal substituiertes Phenyl,
Hal F oder Cl
bedeuten,
umsetzt,
oder
b) falls X H,H bedeutet und R, R¹, R² und n die angegebenen Bedeutungen haben,
eine Verbindung der Formel I, worin X O bedeutet und R, R¹, R² und n die angegebenen Bedeutungen haben, mit komplexen Hydriden unter Aktivierung durch Lewis-saure Metallhalogenide des Typs R'-Al(Cl)₂,
worin
R' A oder Aryl',
A Alkyl mit 1-6 C-Atomen,
Aryl' unsubstituiertes oder ein- oder zweifach durch A', OA' oder Hal substituertes Phenyl,
Hal F oder Cl
bedeuten,
reduziert,
und/oder daß man eine erhaltene Base der Formel I durch Behandeln mit einer Säure in eines ihrer Säureadditionssalze umwandelt.

2. Verfahren nach Anspruch 1, zur Herstellung von Verbindungen der Formel I 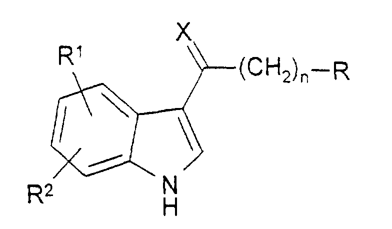 worin
R Hal,
R¹ H,
R² CN
X O oder H,H,
Hal F, Cl, Br oder I und
n 2, 3 oder 4
bedeuten,
sowie ihrer Säureadditionssalze, **dadurch gekennzeichnet, daß** man,
a) falls X O bedeutet und R, R¹, R² und n die angegebenen Bedeutungen haben,
eine Verbindung der Formel II 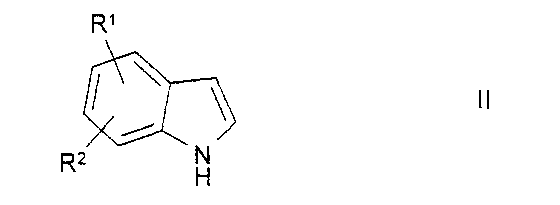 worin
R¹ H und
R² CN,
bedeuten,
mit einer Verbindung der Formel III
R-(CH₂)ₙ-CO-L III
worin
R Hal,
L Cl, Br, I, OH oder eine freie oder reaktionsfähig funktionell abgewandelte OH-Gruppe bedeutet,
Hal F, Cl, Br, I und
n 2, 3 oder 4
bedeuten,
in einer Friedel-Crafts Acylierung unter Katalyse von Lewis-sauren Metallhalogeniden des Typs R'-Al(Cl)₂,
worin
R' A,
A Alkyl mit 1-6 C-Atomen,
bedeuten,
umsetzt,
oder
b) falls X H,H bedeutet und R, R¹, R² und n die angegebenen Bedeutungen haben,
eine Verbindung der Formel I, worin X O bedeutet und R, R¹, R² und n die angegebenen Bedeutungen haben,
mit komplexen Hydriden unter Aktivierung durch Lewis-saure Metallhalogenide des Typs R'-Al(Cl)₂,
worin
R' A,
A Alkyl mit 1-6 C-Atomen,
bedeuten,
reduziert,
und/oder daß man eine erhaltene Base der Formel I durch Behandeln mit einer Säure in eines ihrer Säureadditionssalze umwandelt.

3. Verfahren nach Anspruch 1 oder 2, zur Herstellung von Verbindungen gemäß Formel I, worin X H,H bedeutet und R, R¹, R² und n die angegebenen Bedeutungen haben, **dadurch gekennzeichnet, daß** man die Verbindungen der Formel I, worin X O bedeutet und R, R¹, R² und n die angegebenen Bedeutungen haben, nach Schritt a) herstellt und anschließend nach Schritt b) reduziert.

## Claims

1. Process for the preparation of compounds of the formula I 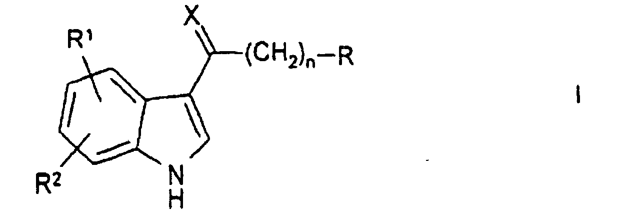 in which
R denotes Hal or methyl,
R¹, R² each, independently of one another, denote H, A', aryl, NH₂, NHA", N(A")₂, COOA"', CN or Hal,
X denotes O or H,H,
A', A", A"' each, independently of one another, denote alkyl having 1-6 C atoms,
Hal denotes F, Cl, Br or I and
n denotes 1, 2, 3, 4, 5 or 6,
and acid-addition salts thereof, **characterised in that**
a) if X denotes O and R, R¹, R² and n have the meanings indicated,
a compound of the formula II 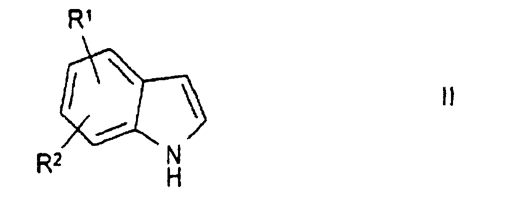 in which
R¹, R² each, independently of one another, denote H, A', aryl, NH₂, NHA", N(A")₂, COOA"', CN or Hal,
A' A", A"' each, independently of one another, denote alkyl having 1-6 C atoms and
Hal denotes F, Cl, Br or I,
is reacted with a compound of the formula III
R-(CH₂)ₙ-CO-L III
in which
R denotes Hal or methyl,
L denotes Cl, Br, I, OH or a free or reactively functionally modified OH group,
Hal denotes F, Cl, Br or I and
n denotes 1, 2, 3, 4, 5 or 6,
in a Friedel-Crafts acylation with catalysis by Lewis-acidic metal halides of the R'-Al(Cl)₂ type,
in which
R' denotes A or aryl',
A denotes alkyl having 1-6 C atoms,
aryl' denotes phenyl which is unsubstituted or mono- or disubstituted by A', OA' or Hal,
Hal denotes F or Cl,
or
b) if X denotes H,H and R, R¹, R² and n have the meanings indicated,
a compound of the formula I in which X denotes O and R, R¹, R² and n have the meanings indicated,
is reduced using complex hydrides with activation by Lewis-acidic metal halides of the R'-Al(Cl)₂ type,
in which
R' denotes A or aryl',
A denotes alkyl having 1-6 C atoms,
aryl' denotes phenyl which is unsubstituted or mono- or disubstituted by A', OA' or Hal,
Hal denotes F or Cl,
and/or **in that** a base of the formula I obtained is converted into one of its acid-addition salts by treatment with an acid.

2. Process according to Claim 1 for the preparation of compounds of the formula I 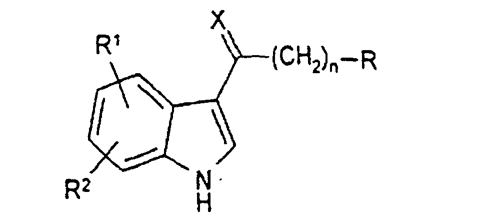 in which
R denotes Hal,
R¹ denotes H,
R² denotes CN,
X denotes O or H,H,
Hal denotes F, Cl, Br or and
n denotes 2, 3 or 4,
and acid-addition salts thereof, **characterised in that**
a) if X denotes O and R, R¹, R² and n have the meanings indicated,
a compound of the formula II 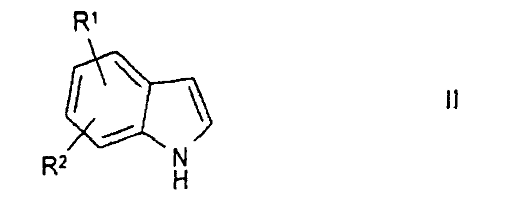 in which
R¹ denotes H and
R² denotes CN,
is reacted with a compound of the formula III
R-(CH₂)ₙ-CO-L III
in which
R denotes Hal,
L denotes Cl, Br, I, OH or a free or reactively functionally modified OH group,
Hal denotes F, Cl, Br, I and
n denotes 2, 3 or 4,
in a Friedel-Crafts acylation with catalysis by Lewis-acidic metal halides of the R'-Al(Cl)₂ type,
in which
R' denotes A,
A denotes alkyl having 1-6 C atoms,
or
b) if X denotes H,H and R, R¹, R² and n have the meanings indicated,
a compound of the formula I in which X denotes O and R, R¹, R² and n have the meanings indicated,
is reduced using complex hydrides with activation by Lewis-acidic metal halides of the R'-Al(Cl)₂ type,
in which
R' denotes A,
A denotes alkyl having 1-6 C atoms,
and/or **in that** a base of the formula I obtained is converted into one of its acid-addition salts by treatment with an acid.

3. Process according to Claim 1 or 2 for the preparation of compounds of the formula I in which X denotes H,H and R, R¹, R² and n have the meanings indicated, **characterised in that** the compounds of the formula I in which X denotes O and R, R¹, R² and n have the meanings indicated are prepared in accordance with step a) and subsequently reduced in accordance with step b).

## Revendications

1. Procédé pour la préparation de composés de la formule I 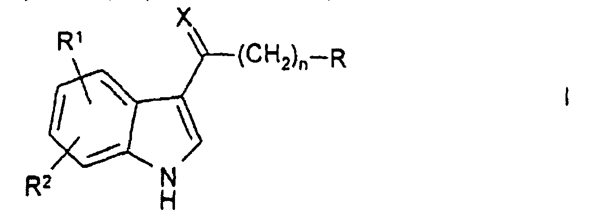 dans laquelle
R représente Hal ou méthyle,
R¹, R² chacun, indépendamment l'un de l'autre, représente H, A', aryle, NH₂, NHA", N(A")₂, COOA"', CN ou Hal,
X représente O ou H,H,
A', A", A"' chacun, indépendamment l'un de l'autre, représente alkyle ayant 1-6 atomes C,
Hal représente F, CI, Br ou I et
n représente 1, 2, 3, 4, 5 ou 6,
et des sels avec addition d'acide afférents, **caractérisé en ce que**
a) si X représente O et R, R¹, R² et n ont les significations indiquées,
un composé de la formule II 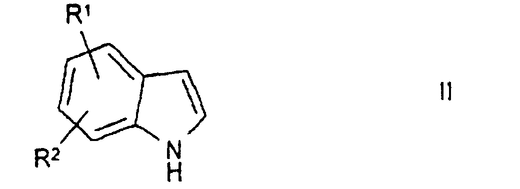 dans laquelle
R¹, R² chacun, indépendamment l'un de l'autre, représente H, A', aryle, NH₂, NHA", N(A")₂, CODA"', CN ou Hal,
A', A", A"' chacun, indépendamment l'un de l'autre, représente alkyle ayant 1-6 atomes C et
Hal représente F, Cl, Br ou I,
est amené à réagir avec un composé de la formule III
R-(CH₂)ₙ-CO-L III
dans laquelle
R représente Hal ou méthyle,
L représente CI, Br, I, OH ou un groupe OH libre ou modifié fonctionnellement de façon réactive,
Hal représente F, CI, Br ou I et
n représente 1, 2, 3, 4, 5 ou 6,
dans une acrylation Friedel-Crafts avec catalyse par halogénures métalliques acides Lewis du type R'-Al(Cl)₂,
selon lequel
R' représente A ou aryle',
A représente alkyle ayant 1-6 atomes C,
aryle' représente phényle qui est non substitué ou mono- ou disubstitué par A', OA' ou Hal,
Hal représente F ou CI,
ou
b) si X représente H,H et R, R¹, R² et n ont les significations indiquées,
un composé de la formule I dans laquelle X représente O et R, R¹, R² et n ont les significations indiquées,
est réduit en utilisant des hydrures complexes avec activation par halogénures métalliques acides Lewis du type R'-Al(Cl)₂,
selon lequel
R' représente A ou aryle',
A représente alkyle ayant 1-6 atomes C,
aryle' représente phényle qui est non substitué ou mono- ou disubstitué par A', OA' ou Hal,
Hal représente F ou CI,
et/ou **en ce qu'**une base de la formule I obtenue est convertie selon l'un de ses sels avec addition d'acide par traitement avec un acide.

2. Procédé selon la revendication 1 pour la préparation de composés de la formule I 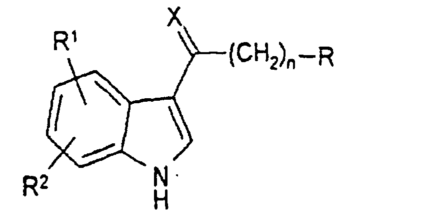 dans laquelle
R représente Hal,
R¹ représente H,
R² représente CN,
X représente O ou H,H,
Hal représente F, Cl, Br ou I et
n représente 2, 3 ou 4,
et des sels avec addition d'acide afférents, **caractérisé en ce que**
a) si X représente O et R, R¹, R² et n ont les significations indiquées,
un composé de la formule II 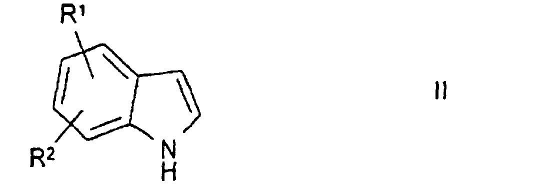 dans laquelle
R¹ représente H et
R² représente CN,
est amené à réagir avec un composé de la formule III
R-(CH₂)ₙ-CO-L III
dans laquelle
R représente Hal,
L représente CI, Br, I, OH ou un groupe OH libre ou modifié fonctionnellement de façon réactive,
Hal représente F, CI, Br, I et
n représente 2, 3 ou 4,
dans une acrylation Friedel-Crafts avec catalyse par halogénures métalliques acides Lewis du type R'-Al(Cl)₂,
selon lequel
R' représente A,
A représente alkyle ayant 1-6 atomes C,
ou
b) si X représente H,H et R, R¹, R² et n ont les significations indiquées,
un composé de la formule I dans laquelle X représente O et R, R¹, R² et n ont les significations indiquées,
est réduit en utilisant des hydrures complexes avec activation par halogénures métalliques acides Lewis du type R'-Al(Cl)₂,
selon lequel
R' représente A,
A représente alkyle ayant 1-6 atomes C,
et/ou **en ce qu'**une base de la formule I obtenue est convertie selon l'un de ses sels avec addition d'acide par traitement avec un acide.

3. Procédé selon la revendication 1 ou 2 pour la préparation de composés de la formule I dans laquelle X représente H,H et R, R¹, R² et n ont les significations indiquées, **caractérisé en ce que** les composés de la formule I dans laquelle X représente O et R, R¹, R² et n ont les significations indiquées sont préparés selon l'étape a) et sont ensuite réduits selon l'étape b).
